# EUROPEAN PATENT SPECIFICATION

(11) **EP 3 824 030 B1**
(45) Date of publication and mention of the grant of the patent: **02.07.2025**
(21) Application number: 19837741.8
(22) Date of filing: 16.07.2019
(51) Int. Cl.: C08L 89/04, C08L 89/06, C14B 7/02, C14B 7/04, C08H 1/06, B32B 9/02, B33Y 70/00, B33Y 80/00

(54) **BIOFABRICATED LEATHER ARTICLES, AND METHODS THEREOF**
BIOHERGESTELLTE LEDERARTIKEL UND VERFAHREN DAFÜR
ARTICLES EN CUIR BIOFABRIQUÉ, ET PROCÉDÉS ASSOCIÉS

(30) Priority: 18.07.2018 US 201862699956 P
(43) Date of publication of application: 26.05.2021
(73) Proprietor: Modern Meadow, Inc., Nutley, New Jersey 07110 (US)
(72) Inventor: SCHACHTSCHNEIDER, Sarah, New York, New York 10002 (US); VARADARAJU, Hemanthram, Jersey City, New Jersey 07306 (US); SCHNEIDER, Morgan, Hillsborough, New Jersey 08844 (US); LEE, Suzanne, Brooklyn, New York 11231 (US); CONGDON, Katherine Amy, Suffolk IP31 IRY (GB); CLAYTON, Callie McBride, Dallas, Texas 75225 (US)
(74) Representative: Maiwald GmbH
(86) International application number: PCT/US2019/041971
(87) International publication number: WO 2020/018516

(56) References cited:
- EP-A1- 1 319 415
- US-A- 4 594 276
- US-A1- 2003 190 438
- US-A1- 2017 233 537
- KOO YOUNGWON ET AL: "3D printed cell-laden collagen and hybrid scaffolds forin vivoarticular cartilage tissue regeneration", JOURNAL OF INDUSTRIAL AND ENGINEERING CHEMISTRY, THE KOREAN SOCIETY OF INDUSTRIAL AND ENGINEERING CHEMISTRY, KOREA, vol. 66, 15 June 2018 (2018-06-15), pages 343 - 355, XP085471029, ISSN: 1226-086X, DOI: 10.1016/J.JIEC.2018.05.049
- SALVATORE, L ET AL.: "Potential of Electrospun Poly(3-hydroxybutyrate)/Collagen Blends for Tissue Engineering Applications", JOURNAL OF HEALTHCARE ENGINEERING, 19 April 2018 (2018-04-19), XP055676618

## Description

### Field of the Invention

The invention herein provides biofabricated leather materials, concentrate comprising collagen that can be used to create biofabricated leather materials, articles comprising biofabricated leather materials, methods for making biofabricated materials, methods for applying biofabricated proteins to substrates and methods for making three dimensional shaped biofabricated materials.

### Background of the Invention

Fabrics are used to make shirts, pants, dresses, skirts, coats, blouses, t-shirts, sweaters, shoes, bags, furniture, blankets, curtains, wall coverings, table cloths, car seats and interiors, and the like. A new biofabricated leather material is taught in co-pending US patent application 15/433,566. The biofabricated collagen solution is well suited for making materials in various shapes and designs as well as for bonding materials together.

### Description of related art

Fabrics with decorations, designs, raised patterns and the like are known. When they are decorated, the decorations maybe stitched on or adhered on to the fabric. Designs are also commonly printed or screen-printed, sprayed, stitched or glued on to fabrics. Designs on fabric typically include letters, words, brand logos, animal shapes, random shapes, geometric shapes and the like.

Co-pending U.S. patent application no. 15/433,693, teaches composite materials and methods for making them. There is a continuing need for new composite materials and methods for making them. There is also a need for a method for transferring protein materials to a substrate and a need for a method for making three dimensional shaped biofabricated materials.

European patent application EP 1 319 415 A1 refers to an adhesion preventive membrane including a nonwoven fabric layer of collagen fibers, having on a surface thereof a coating layer containing a mixture of collagen and hyaluronic acid.

### Summary of the Invention

The present invention according to the claims relates to a method of applying a collagen solution to a substrate, the method including: spraying a collagen solution onto a sheet of acetate forming a collagen coated side of the acetate sheet; drying the collagen; spraying an adhesive onto the collagen; placing a substrate onto the collagen coated side of the acetate sheet; placing the substrate and the collagen coated acetate sheet in a heated press; pressing the substrate and the collagen coated acetate sheet; cooling the substrate and the collagen coated acetate sheet; and removing the acetate sheet to produce a biofabricated material.

Disclosed herein but not claimed is an article, comprising: a biofabricated leather material in a design or pattern and/or a biofabricated material present on a substrate in a design or pattern, such as a lace like design or pattern and/or a raised, for example to impart a three dimensional design or pattern and/or textured surface. The biofabricated material may be adhered to a second material such as a second biofabricated leather material and a fabric. The fabric can be natural, synthetic or both and may be woven, non-woven, a knit and a combination thereof and may have a mesh ranging from 300 threads per square inch to 1 thread per square foot or a pore size greater than or equal to 11µm in diameter.

The biofabricated leather material may comprise recombinant bovine collagen.

Also disclosed herein is an article comprising: one or more materials with opposing edges and a gap between the edges; and a biofabricated leather material that fills the gap and overlaps the opposing edges to bond the opposing edges of the material. The opposing edges that are bonded by the biofabricated leather material are edges of a single continuous material, e.g. the same piece of fabric and/or two or more discontinuous materials, e.g., two separate pieces of fabric, the fabric having the same or substantially the same compositions or different compositions and/or forms, shapes and the like.

The material may be a biofabricated leather material, a fabric, a wood, a wood veneer, a metal, a plastic or a combination thereof. The fabric may be natural, synthetic or a combination thereof and may be woven, non-woven, a knit or a combination thereof and may have a mesh ranging from 300 threads per square inch to 1 thread per square foot or a pore size greater than or equal to 11µm in diameter. The fibers of the fabric may comprise protein, cellulose, or a combination thereof. The edges of the fabric may be devored.

The biofabricated leather material may comprise recombinant bovine collagen.

Another embodiment is an article comprising a material pretreated with a solution; and a biofabricated leather material in a design or pattern bonded to the material. The material may be a cellulose fabric pretreated with a periodate solution and the cellulose fabric may comprise one or more of viscose, acetate, lyocell, bamboo. The periodate pretreatment may include 25% to 100% periodate by weight of the fabric and may be conducted by exposing the fabric to the periodate solution for 15 mins to 24 hours, quenching the periodate with a glycol, e.g., ethylene glycol, propylene glycol, diethylene glycol, dipropylene glycol, triethylene glycol, polyethylene glycol, butylene glycol or a combination thereof, rinsing the fabric with water and drying the fabric.

The fabric may be natural, synthetic or a combination thereof and may be woven, non-woven, a knit or a combination thereof and may have a mesh ranging from 300 threads per square inch to 1 thread per square foot or a pore size greater than or equal to 11µm in diameter.

The fibers of the fabric may comprise protein, cellulose, or a combination thereof. The edges of the fabric may be devored.

The fabric may also be pretreated with a collagen solution such as by applying a 0.5 mg/ml to 10 mg/ml collagen solution to the fabric and/or pouring a collagen solution into a container, cooling the container, mixing the solution, adding a buffer to the solution to induce fibrillation, filtering the solution through the fabric, placing the fabric and the filtrate in the container and mixing.

Methods of making the above articles are also embodied within the present disclosure.

For instance, a method of making a biofabricated leather bonded article is provided, the method comprising: placing a material on a surface; applying an aqueous solution of collagen on the material; and drying to form the biofabricated leather bonded article such as by vacuum, heated air drying, ambient air drying, heated pressing, pressure drying and a combination thereof, the vacuum may be an applied pressure of from 0 to 14 psi and/or the drying may be 30 minutes to 24 hours and/or at a temperature of from about 20°C to 80°C.

Another embodiment of each of the above articles and methods of making may include the use of an aqueous fibrillated cross-linked collagen composition, e.g., a solution, or paste or dough, that may be deposited by pouring, painting, paletting, screen printing, dripping, pipetting, with a nozzle, or other liquid deposition means, e.g., an automated deposition system. Paletting means using a blade or a knife or a flat surface to pick up a paste or dough, transfer it to a substrate such as a textile and spreading it. The composition optionally comprises a binder, for example, binders commonly used in the manufacture of textiles.

Another embodiment of each of the above articles and methods of making may include treating the material onto which the biofabricated material is applied to create one or more holes or voids that may be created by selectively removing a portion of the material physically, chemically, or by burning and such that, for example, the aqueous solution of collagen is applied to the one or more holes or voids.

Another embodiment of each of the above articles and methods of making may include applying the biofabricated material to a rigid substrate. This could include but is not limited to materials such as wood and wood veneers, metals, plastics and the like. The biofabricated material can be used to coat these materials, join them to one another or embed them within the leather itself e.g. marquetry. The biofabricated material may adhere to the rigid substrate on it's own. Alternatively, the biofabricated material may adhere to the rigid substrate with the use of any adhesive.

Also disclosed herein but not claimed is a method of making a biofabricated material, the method including: providing a collagen concentrate; providing a substrate; placing the collagen concentrate on the substrate; placing the substrate with the collagen concentrate in a heated press; pressing and heating the substrate with the collagen concentrate and cooling the substrate with the collagen concentrate to produce a biofabricated material.

Also disclosed herein but not claimed is a method of making a three-dimensional biofabricated material, the method including: applying a solution comprising collagen to a substrate to form a three dimensional shape, optionally repeating the applying; drying the collagen on the substrate, and optionally shaping the three-dimensional shape.

Also disclosed herein but not claimed is a method of making a layered three-dimensional biofabricated material, the method including: forming three dimensional biofabricated materials, attaching the three dimensional biofabricated materials together forming a layered three-dimensional biofabricated material and optionally further shaping the layered three-dimensional biofabricated material.

### Detailed Description of the Invention

The term "collagen" refers to any one of the known collagen types, including collagen types I through XX, as well as to any other collagens, whether natural, synthetic, semi-synthetic, or recombinant. It includes all of the collagens, modified collagens and collagen-like proteins described herein. The term also encompasses procollagens and collagen-like proteins or collagenous proteins comprising the motif (Gly-X-Y)n where n is an integer. It encompasses molecules of collagen and collagen-like proteins, trimers of collagen molecules, fibrils of collagen, and fibers of collagen fibrils. It also refers to chemically, enzymatically or recombinantly-modified collagens or collagen-like molecules that can be fibrillated as well as fragments of collagen, collagen-like molecules and collagenous molecules capable of assembling into a nanofiber.

In some embodiments, amino acid residues, such as lysine and proline, in a collagen or collagen-like protein may lack hydroxylation or may have a lesser or greater degree of hydroxylation than a corresponding natural or unmodified collagen or collagen-like protein. In other embodiments, amino acid residues in a collagen or collagen-like protein may lack glycosylation or may have a lesser or greater degree of glycosylation than a corresponding natural or unmodified collagen or collagen-like protein. Collagen-like proteins also include gelatin.

The collagen in a collagen composition may homogenously contain a single type of collagen molecule, such as 100% bovine Type I collagen or 100% Type III bovine collagen, or may contain a mixture of different kinds of collagen molecules or collagen-like molecules, such as a mixture of bovine Type I and Type III molecules. Such mixtures may include >0%, 10, 20, 30, 40, 50, 60, 70, 80, 90, 95, 99 or <100% of the individual collagen or collagen-like protein components. This range includes all intermediate values. For example, a collagen composition may contain 30% Type I collagen and 70% Type III collagen, or may contain 33.3% of Type I collagen, 33.3% of Type II collagen, and 33.3% of Type III collagen, where the percentage of collagen is based on the total mass of collagen in the composition or on the molecular percentages of collagen molecules.

"Collagen fibrils" are nanofibers composed of tropocollagen (triple helices of collagen molecules). Tropocollagens also include tropocollagen-like structures exhibiting triple helical structures. The collagen fibrils of the invention may have diameters ranging from 1 nm and 1 µm. For example, the collagen fibrils of the invention may have an average or individual fibril diameter ranging from 1, 2, 3, 4, 5, 10, 15, 20, 30, 40, 50, 60, 70, 80, 90, 100, 200, 300, 400, 500, 600, 700, 800, 900, or 1,000 nm (1 µm). This range includes all intermediate values and subranges. In some of the embodiments of the invention collagen fibrils will form networks. Collagen fibrils can associate into fibrils exhibiting a banded pattern and these fibrils can associate into larger aggregates of fibrils. In some embodiments the collagen or collagen-like fibrils will have diameters and orientations similar to those in the top grain or surface layer of a bovine or other conventional leather. In other embodiments, the collagen fibrils may have diameters comprising the top grain and those of a corium layer of a conventional leather.

A "collagen fiber" is composed of collagen fibrils that are tightly packed and exhibit a high degree of alignment in the direction of the fiber. It can vary in diameter from more than 1 µm to more than 10 µm, for example >1, 2, 3, 4, 5, 6, 7, 8, 9, 10, 11, 12 µm or more. Some embodiments of the network of collage fibrils of the invention do not contain substantial content of collagen fibers having diameters greater than 5 µm. The composition of the grain surface of a leather can differ from its more internal portions, such as the corium which contains coarser fiber bundles.

"Fibrillation" refers to a process of producing collagen fibrils. It may be performed by raising the pH or by adjusting the salt concentration of a collagen solution or suspension. In forming the fibrillated collagen, the collagen may be incubated to form the fibrils for any appropriate length of time, including between 1 min and 24 hrs and all intermediate values.

The fibrillated collagen described herein may generally be formed in any appropriate shape and/or thickness, including flat sheets, curved shapes/sheets, cylinders, threads, and complex shapes. These sheets and other forms may have virtually any linear dimensions including a thickness, width or height greater of 10, 20, 30, 40, 50, 60, 70,80, 90 mm; 1, 2, 3, 4, 5, 6, 7, 8, 9, 10, 50, 100, 200, 500, 1,000, 1,500, 2,000 cm or more.

The fibrillated collagen may lack any or any substantial amount of higher order structure. In a preferred embodiment, the collagen fibrils will be unbundled and not form the large collagen fibers found in animal skin and provide a strong and uniform non-anisotropic structure to the biofabricated leather.

In other embodiments, some collagen fibrils can be bundled or aligned into higher order structures. Collagen fibrils in a biofabricated leather may exhibit an orientation index ranging from 0, >0, 0.1, 0.2, 0.3, 0.4, 0.5, 0.6, 0.7, 0.8, 0.9, <1.0, or 1.0, wherein an orientation index of 0 describes collagen fibrils that lack alignment with other fibrils and an orientation index of 1.0 describes collagen fibrils that are completely aligned. This range includes all intermediate values and subranges. Those of skill in the art are familiar with the orientation index which is also incorporated by reference to Sizeland, et al., J. Agric. Food Chem. 61: 887-892 (2013) or Basil- Jones, et al., J. Agric. Food Chem. 59: 9972-9979 (2011).

A biofabricated leather may be fibrillated and processed to contain collagen fibrils that resemble or mimic the properties of collagen fibrils produced by particular species or breeds of animals or by animals raised under particular conditions.

Alternatively, fibrillation and processing conditions can be selected to provide collagen fibrils distinct from those found in nature, such as by decreasing or increasing the fibril diameter, degree of alignment, or degree of crosslinking compared to fibrils in natural leather.

A crosslinked network of collagen, sometimes called a hydrogel, may be formed as the collagen is fibrillated, or it may form a network after fibrillation; in some variations, the process of fibrillating the collagen also forms gel-like network. Once formed, the fibrillated collagen network may be further stabilized by incorporating molecules with di-, tri-, or multifunctional reactive groups that include chromium, amines, carboxylic acids, sulfates, sulfites, sulfonates, aldehydes, hydrazides, sulfhydryls, diazarines, aryl azides, acrylates, epoxides, or phenols.

The fibrillated collagen network may also be polymerized with other agents (e.g. polymers that are capable of polymerizing or other suitable fibers), which could be used to further stabilize the matrix and provide the desired end structure. Hydrogels based upon acrylamides, acrylic acids, and their salts may be prepared using inverse suspension polymerization. Hydrogels described herein may be prepared from polar monomers. The hydrogels used may be natural polymer hydrogels, synthetic polymer hydrogels, or a combination of the two. The hydrogels used may be obtained using graft polymerization, crosslinking polymerization, networks formed of water soluble polymers, radiation crosslinking, and so on. A small amount of crosslinking agent may be added to the hydrogel composition to enhance polymerization.

Average or individual collagen fibril length may range from 100, 200, 300, 400, 500,600, 700, 800, 900, 1,000 (1 µm); 5, 10, 20, 30, 40, 50, 60, 70, 80, 90, 100, 200, 300, 400, 500, 600, 700, 800, 900, 1,000 µm (1 mm) throughout the entire thickness of a biofabricated leather. These ranges include all intermediate values and subranges.

Fibrils may align with other fibrils over 50, 100, 200, 300, 400, 500 µm or more of their lengths or may exhibit little or no alignment. In other embodiments, some collagen fibrils can be bundled or aligned into higher order structures.

Collagen fibrils in a biofabricated leather may exhibit an orientation index ranging from 0, >0, 0.1, 0.2, 0.3, 0.4, 0.5, 0.6, 0.7, 0.8, 0.9, <1.0, or 1.0, wherein an orientation index of 0 describes collagen fibrils that lack alignment with other fibrils and an orientation index of 1.0 describes collagen fibrils that are completely aligned. This range includes all intermediate values and subranges. Those of skill in the art are familiar with the orientation index which is also incorporated by reference to Sizeland, et al., J. Agric. Food Chem. 61: 887-892 (2013) or Basil- Jones, et al., J. Agric. Food Chem. 59: 9972-9979 (2011).

Collagen fibril density of a biofabricated leather may range from about 1 to 1,000 mg/cc, preferably from 5 to 500 mg/cc including all intermediate values, such as 5, 10, 20, 30, 40, 50, 60, 70, 80, 90, 100, 150, 200, 250, 300, 350, 400, 450, 500, 600, 700, 800, 900 and 1,000 mg/cc.

The collagen fibrils in a biofabricated leather may exhibit a unimodal, bimodal, trimodal, or multimodal distribution, for example, a biofabricated leather may be composed of two different fibril preparations each having a different range of fibril diameters arranged around one of two different modes. Such mixtures may be selected to impart additive, synergistic or a balance of physical properties on a biofabricated leather conferred by fibrils having different diameters.

Natural leather products may contain 150-300 mg/cc collagen based on the weight of the leather product. A biofabricated leather may contain a similar content of collagen or collagen fibrils as conventional leather based on the weight of the biofabricated leather, such as a collagen concentration of 100, 150, 200, 250, 300 or 350 mg/cc.

The fibrillated collagen, sometimes called a hydrogel, may have a thickness selected based on its ultimate use. Thicker or more concentrated preparations of the fibrillated collagen generally produce thicker biofabricated leathers. The final thickness of a biofabricated leather may be only 10, 20, 30, 40, 50, 60, 70, 80 or 90% that of the fibril preparation prior to shrinkage caused by crosslinking, dehydration and lubrication.

"Crosslinking" refers to formation (or reformation) of chemical bonds within between collagen molecules. A crosslinking reaction stabilizes the collagen structure and in some cases forms a network between collagen molecules. Any suitable crosslinking agent known in the art can be used including, without limitation, mineral salts such as those based on chromium, formaldehyde, hexamethylene diisocyanate, glutaraldehyde, polyepoxy compounds, gamma 15 irradiation, and ultraviolet irradiation with riboflavin. The crosslinking can be performed by any known method; see, e.g., Bailey et al., Radiat. Res. 22:606-621 (1964); Housley et al., Biochem. Biophys. Res. Commun. 67:824-830 (1975); Siegel, Proc. Natl. Acad. Sci. U.S.A. 71:4826-4830 (1974); Mechanic et al., Biochem. Biophys. Res. Commun. 45:644-653 (1971); Mechanic et al., Biochem. Biophys. Res. Commun. 41:1597-1604 (1970); and Shoshan et al., Biochim. Biophys. Acta 154:261-263 (1968) each of which is incorporated by reference.

Crosslinkers include isocyantes, carbodiimide, poly(aldehyde), poly(azyridine), mineral salts, poly(epoxies), enzymes, thiirane, phenolics, novolac, resole as well as other compounds that have chemistries that react with amino acid side chains such as lysine, arginine, aspartic acid, glutamic acid, hydroxylproline, or hydroxylysine.

A collagen or collagen-like protein may be chemically modified to promote chemical and/or physical crosslinking between the collagen fibrils. Chemical crosslinking may be possible because reactive groups such as lysine, glutamic acid, and hydroxyl groups on the collagen molecule project from collagen's rod-like fibril structure. Crosslinking that involve these groups prevent the collagen molecules from sliding past each other under stress and thus increases the mechanical strength of the collagen fibers. Examples of chemical crosslinking reactions include but are not limited to reactions with the ε-amino group of lysine, or reaction with carboxyl groups of the collagen molecule. Enzymes such as transglutaminase may also be used to generate crosslinks between glutamic acid and lysine to form a stable γ-glutamyl-lysine crosslink. Inducing crosslinking between functional groups of neighboring collagen molecules is known in the art. Crosslinking is another step that can be implemented here to adjust the physical properties obtained from the fibrillated collagen hydrogel-derived materials.

Still fibrillating or fibrillated collagen may be crosslinked or lubricated. Collagen fibrils can be treated with compounds containing chromium or at least one aldehyde group, or vegetable tannins prior to network formation, during network formation, or network gel formation. Crosslinking further stabilizes the fibrillated collagen leather. For example, collagen fibrils pre- treated with acrylic polymer followed by treatment with a vegetable tannin, such as Acacia Mollissima, can exhibit increased hydrothermal stability. In other embodiments, glyceraldehyde may be used as a cross-linking agent to increase the thermal stability, proteolytic resistance, and mechanical characteristics, such as Young's modulus and tensile stress, of the fibrillated collagen.

A biofabricated material containing a network of collagen fibrils may contain 0, >0, 1, 2, 3, 4, 5, 6, 7, 8, 9, 10, 15, 20% or more of a crosslinking agent including tanning agents used for conventional leather. The crosslinking agents may be covalently bound to the collagen fibrils or other components of a biofabricated material or non-covalently associated with them.

Preferably, a biofabricated leather will contain no more than 1, 2, 3, 4, 5, 6, 7, 8, 9 or 10% of a crosslinking agent.

"Lubricating" describes a process of applying a lubricant, such as a fat or other hydrophobic compound or any material that modulates or controls fibril-fibril bonding during dehydration to leather or to biofabricated products comprising collagen. A desirable feature of the leather aesthetic is the stiffness or hand of the material. In order to achieve this property, water-mediated hydrogen bonding between fibrils and/or fibers is limited in leather through the use of lubricants. Examples of lubricants include fats, biological, mineral or synthetic oils, cod oil, sulfonated oil, polymers, organofunctional siloxanes, and other hydrophobic compounds or agents used for fatliquoring conventional leather as well as mixtures thereof. While lubricating is in some ways analogous to fatliquoring a natural leather, a biofabricated product can be more uniformly treated with a lubricant due to its method of manufacture, more homogenous composition and less complex composition.

Other lubricants include surfactants, anionic surfactants, cationic surfactants, cationic polymeric surfactants, anionic polymeric surfactants, amphiphilic polymers, fatty acids, modified fatty acids, nonionic hydrophilic polymers, nonionic hydrophobic polymers, poly acrylic acids, poly methacrylic, acrylics, natural rubbers, synthetic rubbers, resins, amphiphilic anionic polymer and copolymers, amphiphilic cationic polymer and copolymers and mixtures thereof as well as emulsions or suspensions of these in water, alcohol, ketones, and other solvents.

Lubricants may be added to a biofabricated material containing collagen fibrils.

Lubricants may be incorporated in any amount that facilitates fibril movement or that confers leather-like properties such as flexibility, decrease in brittleness, durability, or water resistance. A lubricant content can range from about 0.1, 0.25, 0.5, 1, 2, 3, 4, 5, 6, 7, 8, 9, 10, 15, 20, 25, 30, 35, 40, 45, 50, 55, and 60% by weight of the biofabricated leather.

Other additives may be added to modify the properties of biofabricated leather or material. Suitable additives include but are not limited to dyes, pigments, fragrances, resins, textile binders and microparticles. Resins may be added to modify the stretchability, strength, and softness of the material. Suitable resins include but are not limited to elastomers, acrylic copolymers, polyurethane, and the like. Suitable elastomers include but are not limited to styrene, isoprene, butadiene copolymers such as KRAYTON ^{®} elastomers, Hycar ^{®} acrylic resins. Resins may be used at from about 5% to 200%, or from about 50% to 150% (based on the weight of collagen).

"Dehydrating" or "dewatering" describes a process of removing water from a mixture containing collagen fibrils and water, such as an aqueous solution, suspension, gel, or hydrogel containing fibrillated collagen. Water may be removed by filtration, evaporation, freeze-drying, solvent exchange, vacuum-drying, convection-drying, heating, irradiating or microwaving, or by other known methods for removing water. In addition, chemical crosslinking of collagen is known to remove bound water from collagen by consuming hydrophilic amino acid residues such as lysine, arginine, and hydroxylysine among others. The inventors have found that acetone quickly dehydrates collagen fibrils and may also remove water bound to hydrated collagen molecules. Water content of a biofabricated material or leather after dehydration is preferably no more than 60% by weight, for example, no more than 5, 10, 15, 20, 30, 35, 40, 50 or 60% by weight of the biofabricated leather. This range includes all intermediate values. Water content is measured by equilibration at 65% relative humidity at 25°C and 1 atm.

"Grain texture" describes a leather-like texture which is aesthetically or texturally the similar to the texture of a full grain leather, top grain leather, corrected grain leather (where an artificial grain has been applied), or coarser split grain leather texture. Advantageously, the biofabricated material of the invention can be tuned to provide a fine grain, resembling the surface grain of a leather.

The articles in the invention may include foot wear, garments, gloves, furniture or vehicle upholstery, jewelry and other leather goods and products. It includes but is not limited to clothing, such as overcoats, coats, jackets, shirts, trousers, pants, shorts, swimwear, undergarments, uniforms, emblems or letters, costumes, ties, skirts, dresses, blouses, leggings, gloves, mittens, shoes, shoe components such as sole, quarter, tongue, cuff, welt, and counter, dress shoes, athletic shoes, running shoes, casual shoes, athletic, running or casual shoe components such as toe cap, toe box, outsole, midsole, upper, laces, eyelets, collar, lining, Achilles notch, heel, and counter, fashion or women's shoes and their shoe components such as upper, outer sole, toe spring, toe box, decoration, vamp, lining, sock, insole, platform, counter, and heel or high heel, boots, sandals, buttons, sandals, hats, masks, headgear, headbands, head wraps, and belts; jewelry such as bracelets, watch bands, and necklaces; gloves, umbrellas, walking sticks, wallets, mobile phone or wearable computer coverings, purses, backpacks, suitcases, handbags, folios, folders, boxes, and other personal objects; athletic, sports, hunting or recreational gear such as harnesses, bridles, reins, bits, leashes, mitts, tennis rackets, golf clubs, polo, hockey, or lacrosse gear, chessboards and game boards, medicine balls, kick balls, baseballs, and other kinds of balls, and toys; book bindings, book covers, picture frames or artwork; furniture and home, office or other interior or exterior furnishings including chairs, sofas, doors, seats, ottomans, room dividers, coasters, mouse pads, desk blotters, or other pads, tables, beds, floor, wall or ceiling coverings, flooring; automobile, boat, aircraft and other vehicular products including seats, headrests, upholstery, paneling, steering wheel, joystick or control coverings and other wraps or coverings.

Physical Properties of a biofabricated network of collagen fibrils or a biofabricated leather may be selected or tuned by selecting the type of collagen, the amount of concentration of collagen fibrillated, the degree of fibrillation, crosslinking, dehydration and lubrication.

Many advantageous properties are associated with the network structure of the collagen fibrils which can provide strong, flexible and substantially uniform properties to the resulting biofabricated material or leather. Preferable physical properties of the biofabricated leather according to the invention include a tensile strength ranging from 1, 2, 3, 4, 5, 6, 7, 8, 9, 10, 11, 12, 13, 14, 15 or more MPa, a flexibility determined by elongation at break ranging from 1, 5, 10, 15, 20, 25, 30% or more, softness as determined by ISO 17235 of 4, 5, 6, 7, 8 mm or more, a thickness ranging from 0.2, 0.3, 0.4, 0.5, 0.6, 0.7, 0.8, 0.9, 1.0, 1.1, 1.2, 1.3. 1,4, 1,5, 1.6, 1.7, 1.8, 1.9, 2.0 mm or more, and a collagen density (collagen fibril density) of 10, 20, 30, 40, 50, 60, 70, 80, 90, 100, 200, 300, 400, 500, 600, 700, 800, 900, 1,000 mg/cc or more, preferably 100-500 mg/cc. The above ranges include all subranges and intermediate values.

Thickness. Depending on its ultimate application a biofabricated material or leather may have any thickness. Its thickness preferably ranges from about 0.05 mm to 20 mm as well as any intermediate value within this range, such as 0.05, 0.1, 0.2, 0.5, 1, 2, 3, 4, 5, 6, 7, 8, 9, 10, 11, 12, 13, 14, 15, 16, 17, 18, 19, 20, 25, 30, 40, 50 mm or more. The thickness of a biofabricated leather can be controlled by adjusting collagen content.

Elastic modulus. The elastic modulus (also known as Young's modulus) is a number that measures an object or substance's resistance to being deformed elastically (i.e., non-permanently) when a force is applied to it. The elastic modulus of an object is defined as the slope of its stress-strain curve in the elastic deformation region. A stiffer material will have a higher elastic modulus. The elastic modulus can be measured using a texture analyzer.

A biofabricated leather can have an elastic modulus of at least 100 kPa. It can range from 100 kPa to 1,000 Mpa as well as any intermediate value in this range, such as 0.1, 0.2, 0.3, 0.4, 0.5, 0.6, 0.7, 0.8, 0.9, 1, 2, 3, 4, 5, 6, 7, 8, 9, 10, 50, 100, 200, 300, 400, 500, 600, 700, 800, 900, or 1,000 Mpa. A biofabricated leather may be able to elongate up to 300 % from its relaxed state length, for example, by >0, 1, 2, 3, 4, 5, 6, 7, 8, 9, 10, 20, 30, 40, 50, 60, 70, 80, 90, 100, 150, 200, 250, or 300 % of its relaxed state length.

Tensile strength (also known as ultimate tensile strength) is the capacity of a material or structure to withstand loads tending to elongate, as opposed to compressive strength, which withstands loads tending to reduce size. Tensile strength resists tension or being pulled apart, whereas compressive strength resists compression or being pushed together.

A sample of a biofabricated material may be tested for tensile strength using an Instron machine. Clamps are attached to the ends of the sample and the sample is pulled in opposite directions until failure. Good strength is demonstrated when the sample has a tensile strength of at least 1 Mpa. A biofabricated leather can have a tensile strength of at least 1 kPa. It can range from 1 kPa to 100 Mpa as well as any intermediate value in this range, such as 1, 2, 3, 4, 5, 6, 7, 8, 9, 10, 50, 100, 200, 300, 400, 500kPA; 0.5, 0.6, 0.7, 0.8, 0.9, 1.0, 1.1, 1.2, 1.3, 1.4, 1.5, 2, 3, 4, 5, 6, 7, 8, 9, 10, 20, 30, 40, 50, 60, 70, 80, 90, or 100 Mpa.

Tear strength (also known as tear resistance) is a measure of how well a material can withstand the effects of tearing. More specifically however it is how well a material (normally rubber) resists the growth of any cuts when under tension, it is usually measured in kN/m. Tear resistance can be measured by the ASTM D 412 method (the same used to measure tensile strength, modulus and elongation). ASTM D 624 can be used to measure the resistance to the formation of a tear (tear initiation) and the resistance to the expansion of a tear (tear propagation). Regardless of which of these two is being measured, the sample is held between two holders and a uniform pulling force applied until the aforementioned deformation occurs. Tear resistance is then calculated by dividing the force applied by the thickness of the material. A biofabricated leather may exhibit tear resistance of at least 1, 2, 3, 4, 5, 6, 7, 8, 9, 10, 15, 20, 25, 30, 35, 40, 45, 50, 100, 150 or 200% more than that of a conventional top grain or other leather of the same thickness comprising the same type of collagen, e.g., bovine Type I or Type III collagen, processed using the same crosslinker(s) or lubricants. A biofabricated material may have a tear strength ranging from about 1 to 500 N, for example, 1, 2, 3, 4, 5, 6, 7, 8, 9, 10, 20, 30, 40, 50, 60, 70, 80, 90, 100, 125, 150, 175, 200, 225, 250, 275, 300, 325, 350, 375, 400, 425, 450, 475, or 500 as well as any intermediate tear strength within this range.

Softness. ISO 17235:2015 specifies a non-destructive method for determining the softness of leather. It is applicable to all non-rigid leathers, e.g. shoe upper leather, upholstery leather, leather goods leather, and apparel leather. A biofabricated leather may have a softness as determined by ISO 17235 of 2, 3, 4, 5, 6, 7, 8, 10, 11, 12 mm or more.

Grain. The top grain surface of leather is often regarded as the most desirable due to its soft texture and smooth surface. The top grain is a highly porous network of collagen fibrils.

The strength and tear resistance of the grain is often a limitation for practical applications of the top grain alone and conventional leather products are often backed with corium having a much coarser grain. A biofabricated material as disclosed herein which can be produced with strong and uniform physical properties or increased thickness can be used to provide top grain like products without the requirement for corium backing.

Content of other components. In some embodiments, the collagen is free of other leather components such as elastin or non-structural animal proteins. However, in some embodiments the content of actin, keratin, elastin, fibrin, albumin, globulin, mucin, mucinoids, noncollagen structural proteins, and/or noncollagen nonstructural proteins in a biofabricated leather may range from 0, 1, 2, 3,4, 5, 6, 7, 8, 9 to 10% by weight of the biofabricated leather. In other embodiments, a content of actin, keratin, elastin, fibrin, albumin, globulin, mucin, mucinoids, noncollagen structural proteins, and/or noncollagen nonstructural proteins may be incorporated into a biofabricated leather in amounts ranging from >0, 1, 2, 3,4, 5, 6, 7, 8, 9, 10, 11, 12, 13, 14, 15, 16, 17, 18, 19, 20% or more by weight of a biofabricated leather. Such components may be introduced during or after fibrillation, cross-linking, dehydration or lubrication.

A "leather dye" refers to dyes which can be used to color leather or biofabricated leather.

These include acidic dyes, direct dyes, lakes, sulfur dyes, basic dyes and reactive dyes. Dyes and pigments can also be incorporated into a precursor of a biofabricated leather, such as into a suspension or network gel comprising collagen fibrils during production of the biofabricated leather.

"Fillers". In some embodiments a biofabricated leather may comprise fillers, other than components of leather, such as microspheres. One way to control the organization of the dehydrated fibril network is to include filling materials that keep the fibrils spaced apart during dehydration. These filler materials include nanoparticles, microparticles, or various polymers such as syntans commonly used in the tanning industry. These filling materials could be part of the final dehydrated leather material, or the filling materials could be sacrificial, that is they are degraded or dissolved away leaving open space for a more porous fibril network. The shape and dimension of these fillers may also be used to control the orientation of the dehydrated fibril network.

In some embodiments a filler may comprise polymeric microsphere(s), bead(s), fiber(s), wire(s), or organic salt(s). Other materials may also be embedded or otherwise incorporated into a biofabricated leather or into a network of collagen fibrils according to the invention. These include, but are not limited to one fibers, including both woven and nonwoven fibers as well as cotton, wool, cashmere, angora, linen, bamboo, bast, hemp, soya, seacell, fibers produced from milk or milk proteins, silk, spider silk, other peptides or polypeptides including recombinantly produced peptides or polypeptides, chitosan, mycelium, cellulose including bacterial cellulose, wood including wood fibers, rayon, lyocell, vicose, antimicrobial yarn (A.M.Y.), Sorbtek, nylon, polyester, elastomers such as lycra^{®}, spandex or elastane and other polyester-polyurethane copolymers, aramids, carbon including carbon fibers and fullerenes, glass including glass fibers and nonwovens, silicon and silicon-containing compounds, minerals, including mineral particles and mineral fibers, and metals or metal alloys, including those comprising iron, steel, lead, gold, silver, platinum, copper, zinc and titanium, which may be in the form of particles, fibers, wires or other forms suitable for incorporating into biofabricated leather. Such fillers may include an electrically conductive material, magnetic material, fluorescent material, bioluminescent material, phosphorescent material or other photoluminescent material, or combinations thereof. Mixtures or blends of these components may also be embedded or incorporated into a biofabricated leather, for example, to modify the chemical and physical properties disclosed herein.

Various forms of collagen are found throughout the animal kingdom. The collagen used herein may be obtained from animal sources, including both vertebrates and invertebrates, or from synthetic sources. Collagen may also be sourced from byproducts of existing animal processing. Collagen obtained from animal sources may be isolated using standard laboratory techniques known in the art, for example, Silva et. Al., Marine Origin Collagens and its Potential Applications, Mar. Drugs, 2014 Dec., 12(12); 5881-5901).

The collagen described herein also may be obtained by cell culture techniques including from cells grown in a bioreactor.

Collagen may also be obtained via recombinant DNA techniques. Constructs encoding non-human collagen may be introduced into host organisms to produce non-human collagen. For instance, collagen may also be produced with yeast, such as Hansenula polymorpha, Saccharomyces cerevisiae, Pichia pastoris and the like as the host. Further, in recent years, bacterial genomes have been identified that provide the signature (Gly-Xaa-Yaa)n repeating amino acid sequence that is characteristic of triple helix collagen. For example, gram positive bacterium Streptococcus pyogenes contains two collagen-like proteins, Scl1 and Scl2 that now have well characterized structure and functional properties. Thus, it would be possible to obtain constructs in recombinant E. coli systems with various sequence modifications of either Scl1 or Scl2 for establishing large scale production methods. Collagen may also be obtained through standard peptide synthesis techniques. Collagen obtained from any of the techniques mentioned may be further polymerized. Collagen dimers and trimers are formed from self-association of collagen monomers in solution.

Materials that are useful in the present invention include but are not limited to biofabricated leather materials natural or synthetic woven fabrics, non-woven fabrics, knitted fabrics, mesh fabrics, and spacer fabrics.

Any material that retains the collagen fibrils can be useful in the present invention. In general, fabrics that are useful have a mesh ranging from 300 threads per square inch to 1 thread per square foot or a pore size greater than or equal to about 11µm in diameter. Spun lace materials may also be useful. In some embodiments, water soluble fabrics are useful. When utilized, the portion of the fabric exposed to the solution of collagen dissolves forming a void or hole in the fabric, and the collagen fills the void or hole. Water soluble fabrics are typically formed from polyvinyl alcohol fibers and coated with a resin such as polyvinyl alcohol, polyethylene oxide, hydroxyalkylcellulose, carboxymethylcellulose, polyacrylamide, polyvinyl pyrrolidone, polyacrylate and starch. Alternatively, the void or hole may be covered with a secondary material such as, natural or synthetic woven fabrics, non-woven fabrics, knitted fabrics, mesh fabrics, and spacer fabrics.

Alternatively, biofabricated leather material may be used to plug a void or hole cut into fabric. The size of the void or hole may vary depending on the design to be imparted. The shape of the void or hole may vary depending on the design. Suitable dimensions of void or holes may range from about 0.1 inches to about 5 meters. Suitable shapes include but are not limited to circles, squares, rectangles, triangles, elliptical, ovals and brand logos.

Some materials lend themselves to pretreatment to improve bonding of biofabricated leather materials. Pretreatment may include collagen coating, resin coating, devore of the fabric (also known as burn-out method), chemical or combinations thereof. For example, a chemical pretreatment for materials made from cellulose fibers, may include periodate (an oxidant) solution treatment. Suitable cellulose fabrics are selected from the group consisting of viscose, acetate, lyocell, bamboo and combinations thereof. The oxidant opens sugar rings in the cellulose and enable the collagen to bind to the open rings. The concentration of the oxidant in the solution depends on the extent of oxidation desired. In general, higher the concentration of oxidant or longer the reaction time, higher degree of oxidation is achieved. In an embodiment of the present invention, the oxidation reaction may be carried out for a desired amount of time to achieve the desired level of oxidation. The oxidation reaction can be carried out at various temperatures, depending on the type of oxidant used. The inventors have preferred using controlled oxidation at room temperature over a time range of 15 minutes-24 hours. The amount of sodium periodate ranges from 25% to 100% offers on weight of the fabric. As used herein, offer means the amount of an additive based on the weight % of collagen. Other chemical pretreatments are taught in Bioconjugate Techniques by Greg Hermanson, which is hereby incorporated by reference.

The biofabricated leather solution as described herein may include any appropriate non-human collagen source and/or combination as discussed herein.

As an initial step in the formation of the collagen materials described herein, the starting collagen material may be placed in solution and fibrillated. The collagen concentration may range from approximately 0.5 g/L to 10 g/L. Collagen fibrillation may be induced through the introduction of salts to the collagen solution. The addition of a salt or a combination of salts such as sodium phosphate, potassium phosphate, potassium chloride, and sodium chloride to the collagen solution may change the ionic strength of the collagen solution. Collagen fibrillation may occur as a result of increasing electrostatic interactions, through greater hydrogen bonding, Van der Waals interactions, and covalent bonding. Suitable salt concentrations may range, for example, from approximately 10 mM to 5M.

A collagen network may also be highly sensitive to pH. During the fibrillation step, the pH may be adjusted to control fibril dimensions such as diameter and length. Suitable pH may range from approximately 5.5 to 10. After the fibrillation step prior to filtration, the pH of the solution is adjusted to a pH range from approximately 3.5 to 10, for example 3.5 to 7, or 3.5 to 5.

The overall dimensions and organization of the collagen fibrils will affect the toughness, stretch- ability, and breathability of the resulting fibrillated collagen derived materials. This may be of use for fabricating fibrillated collagen derived leather for various uses that may require different toughness, flexibility, and breathability.

One way to control the organization of the dehydrated fibril network is to include filling materials that keep the fibrils spaced apart during drying. These filler materials could include nanoparticles, microparticles, microspheres, microfibers, or various polymers commonly used in the tanning industry. These filling materials could be part of the final dehydrated leather material, or the filling materials could be sacrificial, that is they are degraded or dissolved away leaving open space for a more porous fibril network.

The collagen or collagen-like proteins may be chemically modified to promote chemical and physical crosslinking between the collagen fibrils. Collagen-like proteins were taught in the United States patent application US 2012/0116053 A1, which is hereby incorporated by reference. Chemical crosslinking may be possible because reactive groups such as lysine, glutamic acid, and hydroxyl groups on the collagen molecule project from collagen's rod-like fibril structure. Crosslinking that involve these groups prevent the collagen molecules from sliding past each other under stress and thus increases the mechanical strength of the collagen fibers. Examples of chemical crosslinking reactions include but are not limited to reactions with the ε-amino group of lysine, or reaction with carboxyl groups of the collagen molecule.

Enzymes such as transglutaminase may also be used to generate crosslinks between glutamic acid and lysine to form a stable γ-glutamyl-lysine crosslink. Inducing crosslinking between functional groups of neighboring collagen molecules is known in the art. Crosslinking is another step that can be implemented here to adjust the physical properties obtained from the fibrillated collagen hydrogel-derived materials.

Once formed, the fibrillated collagen network may be further stabilized by incorporating molecules with di-, tri-, or multifunctional reactive groups that include chromium, amines, carboxylic acids, sulfates, sulfites, sulfonates, aldehydes, hydrazides, sulfhydryls, diazarines, aryl-, azides, acrylates, epoxides, or phenols.

The fibrillated collagen network may also be polymerized with other agents (e.g. polymers that are capable of polymerizing or other suitable fibers) that form a hydrogel or have fibrous qualities, which could be used to further stabilize the matrix and provide the desired end structure. Hydrogels based upon acrylamides, acrylic acids, and their salts may be prepared using inverse suspension polymerization. Hydrogels described herein may be prepared from polar monomers. The hydrogels used may be natural polymer hydrogels, synthetic polymer hydrogels, or a combination of the two. The hydrogels used may be obtained using graft polymerization, crosslinking polymerization, networks formed of water soluble polymers, radiation crosslinking, and so on. A small amount of crosslinking agent may be added to the hydrogel composition to enhance polymerization.

The viscosity of the collagen solution can range from 1 cP to 10000 cP at 20°C depending on the concentration of the collagen solution, instrument, spindle or speed used. Using a Brookfield Viscometer with Spindle 62 at 6 rpm a 10g/L collagen solution may measure approximately 2565 cP and at 3 rpm a 10g/L collagen solution may measure approximately 3300 cP. The solutions can be poured, sprayed, painted, or applied to a surface. The viscosity may vary depending on how the final material is formed. Where a higher viscosity is desired, known thickening agents such as carboxymethylcellulose and the like can be added to the solution. Alternatively, the amount of collagen in the solution can be adjusted to vary the viscosity. The viscosity of collagen concentrate could range from 10,000 cP to 300,000 cP.

The flexibility in the collagen solution enables the production of new materials made entirely through the deposition of said collagen solution, for example the creation of biofabricated leather lace materials or 3-dimensional materials. In a sense, the collagen solution can be poured, pipetted, dripped, sprayed through a nozzle, painted or paletted, screen printed, or robotically applied or dip a secondary material into the collagen solution. A textured surface can be achieved through utilizing an apertured material in the formation process of the material. The collagen composition also enables the use of masking, stenciling and molding techniques. The application of the biofabricated leather solution also enables modifying the properties of the material to which it is applied. For example, the biofabricated leather solution can make the end material stronger, more supple, more rigid, more flexible, more elastic or softer.

In one embodiment, the collagen solution is filtered to remove water and create a concentrate. By concentrate is meant a viscous flowable material. The concentrate contains from 5% to 30% by weight of fibrillated collagen. In this state, the collagen can be mixed with other materials providing different properties than from the collagen solution. For example, the concentrate can be mixed in extruders such as single screw or twin screw extruders. Materials that can be mixed with the concentrate in the extruder include resins, cross-linkers, dyes or pigments, fat-liquors, fibers, binders, microspheres fillers and the like. Due to its higher viscosity the concentrate can be applied to different substrates with different techniques, for example palleting. The same materials can be mixed with conventional techniques as well. The amounts of binder or adhesive can range from 1:3 to 1:1 (binder/adhesive to collagen). The concentrate at the lower end of the weight of fibrillation collagen range may be filtered and dried and the concentrate at the higher end of the weight of fibrillation collagen range may be dried.

As mentioned, a biofabricated leather material derived from the methods described above may have similar gross structural and physical characteristics as leathers produced from animal 5 hides. In general, the biofabricated leather materials described herein may be derived from sources other than sheets or pieces of animal hide or skin, although animal hide or skin may be the source of the collagen used in preparing the fibrillated collagen. The source of the collagen or collagen-like proteins may be isolated from any animal (e.g. mammal, fish), or more particularly cell/tissue cultured, source (including in particular microorganism).

The biofabricated leather material may include agents that stabilize the fibril network contained therein or may contain agents that promote fibrillation. As mentioned in previous sections, cross-linking agents (to provide further stability), nucleating agents (to promote fibrillation), and additional polymerizing agents (for added stability) may be added to the collagen solution prior to fibrillation (or after) to obtain a fibrillated collagen material with desired characteristics (e.g. strength, bend, stretch, and so forth).

As mentioned, following dehydration or drying, the engineered biofabricated leather materials derived from the methods discussed above have a water content of less than 20% by weight. The water content of the engineered biofabricated leather materials may be fine-tuned in the finishing steps to obtain leather materials for differing purposes and desired characteristics.

As mentioned, any of these biofabricated leathers may be tanned (e.g., using a tanning agent including vegetable (tannins), chromium, alum, zirconium, titanium, iron salts, or a combination thereof, or any other appropriate tanning agent). Thus, in any of the resulting biofabricated leather materials described herein, the resulting material may include a percent (e.g., between 0.01% and 10%) of a residual tanning agent (e.g. tannin, chromium, etc.). Thus, the collagen fibrils in the resulting biofabricated leather material are modified to be tanned, e.g., cross-linked to resist degradation.

The biofabricated leather materials may be treated to provide surface textures. Suitable treatments include but are not limited to embossing, debossing, filling in of molds, coating of textured surfaces, and vacuum forming with an apertured plate below the material. As is known in the art, the pressure and temperature at which the embossing and debossing are performed may vary depending on the desired texture and design. Surface coating and finishes known in the leather industry may be applied to the biofabricated leather materials. Alternatively, a textured surface may be created using the concentrate by applying the concentrate on a surface and using a tool such as a toothpick, a needle, and the like to pull peaks.

As mentioned above, in any of the variations for making the biofabricated leathers described herein, the material could be tanned (cross-linked) as the collagen is fibrillated and/or separately after fibrillation has occurred, prior to dehydration. For example, tanning may include crosslinking using an aldehyde (e.g., Glutaraldehyde) and/or any other tanning agent. Thus in general a tanning agent includes any collagen fibril cross-linking agent such as aldehydes cross linkers, chromium, amine, carboxylic acid, sulfate, sulfite, sulfonate, aldehyde, hydrazide, sulfhydryl, diazirine,

Some methods for making a material including a biofabricated leather material include providing a material, pretreating the material to make it suitable for bonding with collagen, applying collagen solution to the material and drying. Drying may include removing water through a vacuum, heated air drying, ambient air drying, heated pressing and pressure drying. Where pretreatment is required, the pretreatment is either cutting voids or holes into the material, chemically removing certain fibers and treating the material with a chemical or collagen solution. Other methods do not require a pretreatment of the material. Where pretreatment is not required, the material is either partially water soluble or retains collagen but allows water to pass through. Suitable mesh sizes range from 300 threads per square inch to 1 thread per square foot. As used herein, the term bonded or bonding to the fabric mean attached such that the biofabricated leather does not easily peel away from the fabric when pulled by hand. A suitable method for testing the efficacy of bonding is a peel strength test performed on an instrument such as an Instron material testing machine. Jaws of the machine are attached to the biofabricated leather material and the material which it was bonded to, and the jaws are pulled apart until the materials tear or separate. The force to tear is reported in N/mm. Suitable peel strengths range from about 0.5 N/mm to 100 N/mm, inclusive of all values and ranges there between, for example, 1, 2, 3, 5, 7.5, 9, 10, 12.5, 15, 18.75, 20, 21, 24.5, 30, 33.25, 35, 40, 42.5, 47.75, 50, 55, 60, 65, 70, 75, 79, 80, 85, 90, 91, 92, 93, 94, and 95.

In one embodiment a method of making a biofabricated material, including: providing a collagen concentrate; providing a substrate; placing the collagen concentrate on the substrate; placing the substrate with the collagen concentrate in a heated press; pressing and heating the substrate with the collagen concentrate and cooling the substrate with the collagen concentrate to produce a biofabricated material is taught. The total solids in the collagen concentrate may range from about 2.5% to about 12% based on the total weight of the concentrate. The substrate may be selected from the group consisting of fabrics, wood, porous plastic, 3D printed structures, yarns, fibers and the like. The collagen concentrate may be placed on the substrate by pouring, spraying, paletting, depositing and the like. The collagen concentrate may be evenly spread over the substrate by using a draw down bar, a roller, a mold, a knife and the like. The concentration of collagen in the concentrate may range from about 5 g/L to about 30 g/L. The ratio of the collagen concentrate to the substrate may range from about 80/20 to about 40/60. The collagen concentrate is pressed onto the substrate using a heated press. Suitable heated presses include but are not limited to those that are commercially available from Carver, Cartigliano and the like. The temperature on the heated press may range from about 25°C to about 190°C. The pressure on the heated press may range from about 0.2 to about 4 metric tons. The pressing time may range from about 10 seconds to about 15 minutes. Suitable cooling methods include but are not limited to water cooling, air cooling, fans and the like.

In one embodiment a method of making a biofabricated material is provided. A collagen concentrate is provided to a substrate, e.g., onto a surface of the substrate. The substrate is heated with the collagen concentrate, for instance with a heated press, optionally with pressing (prior, after, and/or simultaneously with the heating. The heated substrate can then be allowed to cool to ambient temperature or further/expedited cooling, for instance in a cooling apparatus.

Like other embodiments disclosed herein, recombinant bovine collagen is preferred as the collagen concentrate and various substrates as disclosed herein may be used, e.g., fabrics, wood, porous plastic, 3D printed structures, yarns and fibers.

In one embodiment, the ratio of collagen concentrate to substrate is from about 80/20 to 40/60 based on weight, inclusive of 70/30, 30/70, 60/40, 50/50 and all ranges and values therebetween. If applied, the pressure, for instance, with the heated press ranges from 0.2 to 4 metric tons, including 1, 1.5, 1.75, 2, 2.5, 3, and 3.5, including all ranges and values therebetween.

The temperature at which the substrate comprising the collagen concentrate is heated is for example from 25 to 190°C, including all ranges and values therebetween, and is heated (and optionally pressed) for 10 seconds to 15 minutes, for example. When heated, collagen may be denatured into gelatin.

In one embodiment a method of making a biofabricated material, a collagen solution is sprayed onto a sheet of acetate forming a collagen coated side of the acetate sheet, the coated side being a complete coating or partial coating, e.g., 10, 15, 20, 30, 40, 50, 60 or 75% coating of the acetate sheet. Thereafter, the collagen is dried.

The dried collagen film can then be transferred to a substrate by applying the acetate sheet including the dried collagen film to the substrate and applying heat, optionally pressure simultaneously, in a heated press, as an example. Preferably, prior to applying the collagen sheet to the substrate, an adhesive is sprayed onto the collagen to facilitate transference of the collagen. The substrate is then cooled.

Like other embodiments, the collagen solution preferably comprises recombinant bovine collagen.

The optional use of the adhesive is preferably one comprised of polyurethanes, acrylics, epoxies and combinations thereof.

Like other embodiments disclosed herein, recombinant bovine collagen as the collagen concentrate is preferred and various substrates as disclosed herein may be used, e.g., fabrics, metals, wood, plastics, yarn, fibers, 3D printed structures, walls, concrete, skin and leather.

If applied, the pressure, for instance, with the heated press ranges from 1 to 11 metric tons, including 2, 5, 7, 10 and all values and ranges therebetween.

The temperature at which the substrate is heated is for example from 25 to 190°C, including 50, 75, 100, 125, 150, 175 and all values and ranges therebetween for a time, including the optional pressing, from 1 to 30 mins, and all values and ranges therebetween.

In one embodiment a method of making a solid biofabricated three-dimension material involves applying a solution comprising collagen to a substrate to form a three dimensional shape. The application of the collagen solution can be repeated once or any number of times to build up collagen material into target dimensions of the three dimensional shape. After each application, the collagen solution may be allowed to air dry or may be dried in or with a drying device, e.g., warm air, oven etc. The collagen solution can also be dried after all of the collagen solutions that are going to be applied have been put on the substrate or in the case of several applications of collagen solutions to the substrates, instead of drying after each application, drying can be effectuated at intermediate stages of the application step(s), e.g. in the case of three applications, the drying can be done after the second application but prior to the third application. After the drying the collagen solution, the substrate may be removed prior to forming a three dimensional material. Alternatively, the three dimensional material is formed on the substrate.

In one aspect of this embodiment, the shape of collagen that is now dried on the substrate can be shaped into any desired three dimensional shape, e.g., with customary fabric machining tools, other shaping tools, lasers and the like.

Like other embodiments disclosed herein, recombinant bovine collagen as the collagen concentrate is preferred and various substrates as disclosed herein may be used, e.g., fabrics, wood, porous plastic, 3D printed structures, yarns and fibers.

The substrate can be coated with the collagen solution completely or partially, e.g., 10, 15, 20, 30, 40, 50, 60 or 75% coating of the substrate.

The following examples are for illustrative purposes. The claims should not be limited to the details therein.

### Example 1

A piece of fabric (3" in diameter, of Lyocell 50% & Organic Cotton 50%, purchased from Simplifi Fabric). The fabric was placed in a Buchner funnel having a diameter of 3 inches.

The Buchner funnel had a filter paper of (Whatman Grade 1 filter paper, purchased from Sigma Aldrich). A solution (75 mL) of fibrillated, cross-linked, and fat liquored collagen was poured onto the fabric and filter paper and vacuum was applied (25 in Hg). The fabric and biofabricated material was removed from the Buchner funnel and dried for 24 hours in room temperature. The biofabricated leather was bonded onto the surface of the fabric such that it did not easily peel away from the fabric when pulled by hand.

The fibrillated, cross-linked, and fat liquored collagen solution was made by dissolving collagen in 0.1N HCl at 10 g/L and was stirred at 350 rpm for 4 hours. The pH was adjusted to 7.2 by adding 1 part 10x PBS to 9 parts collagen by weight and the solution was stirred at 350 rpm for 4 hours. 10% gluteraldehyde (by weight of collagen) was added and mixed for one hour. Then 100% Hycar26652 (by weight of collagen) was added and mixed for 30 mins. 50% truposol BEN (by weight of collagen) was added and mixed for 30 mins. And 10% black pigment (by weight of collagen) was added and mixed for 30 mins. Lastly, the pH was adjusted to 4 with formic acid.

### Example 2

A piece of fabric (12" x 12", of Lyocell 50% & Organic Cotton 50%, purchased from Simplifi Fabric) was placed on a surface and a circle was cut into the fabric using scissors. The diameter of the circle was 3 inches. The fabric was placed in a vacuum filter having a diameter of 4.5 inches. The vacuum filter had a filter paper of (Whatman Grade 589/2 filter paper, purchased from Sigma Aldrich). A solution (250 grams) of fibrillated, cross-linked, and fat liquored collagen from Example 1 was poured onto the fabric and filter paper and pressure vacuum was applied (50 psi). The fabric and biofabricated material were removed from the Buchner funnel and dried for 24 hours at room temperature. The biofabricated leather filled the 3" hole and was bonded to the fabric at the edges of the hole such that it did not easily peel away from the fabric when pulled by hand.

### Example 3

A piece of fabric (12" x 12" Cotton, purchased from Whaleys of Bradford) was placed on a surface and a circle was cut into the fabric using scissors. The diameter of the circle was 3 inches. A piece of water dissolvable fabric (Dissolvable A4954, purchased from Whaleys of Bradford) was then placed on a surface and a circle was cut out of the fabric. The diameter of the circle was 3.5 inches. The circle of dissolvable fabric was then glued over the hole in the cotton fabric using a holt melt adhesive (Spunfab POF4700). The combined fabrics were then placed in a vacuum filter having a diameter of 4.5 inches. The filter had a filter paper of (Whatman Grade 589/2 filter paper, purchased from Sigma Aldrich). A solution (250g) of fibrillated, cross-linked, and fat liquored collagen from Example 1 was poured onto the fabric and filter paper and pressure vacuum was applied (50psi). The dissolvable fabric that was in contact with the collagen solution dissolved leaving a hole with the biofabricated material filling the hole. The fabric and biofabricated material were removed from the filter and dried for 24 hours at room temperature. The biofabricated leather was bonded to the fabric such that it did not easily peel away from the fabric when pulled by hand.

### Example 4

Two pieces of fabric (Lyocell 50% & Organic Cotton 50%, purchased from Simplifi Fabric) each having a length of 2 feet and a width of 2 feet are placed on a surface. The two edges of each piece of material were brought into a 1inch gap and held in place on a large Buchner funnel with a filter paper underneath the fabric. A solution of fibrillated, cross-linked, and fat liquored collagen from Example 1 was poured onto the fabric and filter paper and vacuum was applied. The fabric and biofabricated material were removed from the Buchner funnel and dried for 24 hours at room temperature. The biofabricated leather was bonded to the fabric such that it did not easily peel away from the fabric when pulled by hand. The fabric was bonded sufficiently to replace a stitch.

### Example 5

Two pieces of fabric (polycotton, devoré fabric, purchased from Whaleys of Bradford) each having a length of 8" and a width of 4" were placed on a surface. A long edge of each piece of fabric had ½ an inch devoréd leaving behind only the polyester fibers. The two devoréd edges of the material were brought to within 1 inch of each other and held in place on a large Buchner funnel with a filter paper underneath the fabric. A solution of fibrillated, cross-linked, and fat liquored collagen from Example 1 was poured onto the fabric and filter paper and vacuum (10 inHg) was applied. The fabric and biofabricated material were removed from the Buchner funnel and dried for 1 hour at 50°C in a dehydrator. The biofabricated leather was bonded to the fabric such that it did not easily peel away from the fabric when pulled by hand. The fabric was bonded sufficiently to replace a stitch.

### Example 6

Pieces of cellulose fabric (3" diameter circles, Lyocell 50% & Organic Cotton 50%, purchased from Simplifi Fabric) were treated with a sodium periodate solution. Various amounts of sodium periodate (25% to 100% offers on weight of fabric) were dissolved in 200 mL distilled water and the fabric was added and mixed overnight. The next morning, the fabric was quenched using ethylene glycol (10mL), rinsed with cold water and dried. The fabric was placed in a Buchner funnel having a diameter of 3 inches. The Buchner funnel had a filter paper of Whatman Grade 1 filter paper, purchased from Sigma Aldrich). A solution (75 mL) of fibrillated, cross-linked, and fat liquored collagen from Example 1 was poured onto the fabric and filter paper and vacuum was applied (25 inHg). The fabric and biofabricated material were removed from the Buchner funnel and dried for 24 hours at room temperature. The biofabricated leather was bonded to the fabric such that it did not easily peel away from the fabric when pulled by hand.

### Example 7

A piece of cellulose fabric (4" x 4", Lyocell 50% & Organic Cotton 50%, purchased from Simplifi Fabric) was treated with a sodium periodate solution. Sodium periodate (a 25% offer on the weight of fabric) was dissolved in 200 mL distilled water and the fabric was added and mixed overnight. The next morning, the fabric was quenched using ethylene glycol (10mL) rinsed with cold water and dried. Next a 0.5mg/ml collagen solution (250mL) was poured into a beaker and the beaker was placed in an ice bath on a mixing plate. A sodium phosphate buffer (27.7 mL, 0.2M sodium phosphate buffer, the buffer was made by mixing 54.7 mL of phosphoric acid, Sigma Aldrich, with 3945.3 mL of deionized water and then the pH was raised to 11.2 using 10M sodium hydroxide, VWR) was added to the collagen solution to start the fibrillation process. The solution was then filtered with the dried fabric in a 90mm Buchner funnel. The filtrate, along with the fabric, were placed back into the beaker and mixed for 3 hours. After the 3 hours, the fabric was removed from the filtrate. A solution of fibrillated, cross-linked, and fat liquored collagen from Example 1 was poured into a metal spray atomizer (purchased on amazon.com). This solution was sprayed directly onto the surface of the fabric, a company logo shaped stencil was used to control where the collagen solution was applied. The sample was dried in a tunnel dryer, at 50°C, for 30 mins and then air dried for 2 hours. The biofabricated leather was bonded to the fabric such that it did not easily peel away from the fabric when pulled by hand.

### Example 8

A piece of fabric (12" x 12" polycotton, devoré fabric, purchased from Whaleys of Bradford) had a 4" circle coated with devoré paste (sourced from Dharma Trading), this was then allowed to dry before being heated to 360°F for 1 min 30 secs, the fabric was then washed under the tap to remove all of the burnt cotton fibres from the circle. The fabric was then placed in a vacuum filter having a diameter of 4.5 inches. Underneath the fabric was a perforated metal sheet (1/32 of an inch in thickness) with small holes of 1/8 of an inch in diameter, and underneath this was a filter paper of (Whatman Grade 589/2 filter paper, purchased from Sigma Aldrich). A solution (250g) of fibrillated, cross-linked, and fat liquored collagen from Example 1 was poured onto the fabric and pressure vacuum was applied (50 psi). The metal grid underneath the fabric, combined with the vacuum, caused a three dimensional surface texture to be created through the process of applying the biofabricated leather material to the surface of the fabric. The fabric and biofabricated material were removed from the filter and dried for 24 hours at room temperature. The biofabricated leather was bonded to the fabric such that it did not easily peel away from the fabric when pulled by hand, and after visual inspection was shownto be integrated into the fabric structure itself.

### Example 9

A piece of fabric (3" in diameter, of Lyocell 50% & Organic Cotton 50%, purchased from Simplifi Fabric) was placed in a Buchner funnel having a diameter of 3 inches. The Buchner funnel had a filter paper of (Whatman Grade 1 filter paper, purchased from Sigma Aldrich). A stencil in the shape of the company name, with a 3 inch diameter, was placed on top of the fabric and a solution (75mL) of fibrillated, cross-linked, and fat liquored collagen from Example 1 was poured onto the fabric inside the stencil and vacuum was applied (25 inHg). The fabric and biofabricated material were removed from the Buchner funnel, the stencil was removed and the material was dried for 24 hours at room temperature. Due to the stencil, the application of the biofabricated leather material was controlled to specific areas on the fabric.

The biofabricated leather was bonded to the fabric such that it did not easily peel away from the fabric when pulled by hand.

### Example 10

A 4" plastic embroidery hoop (purchased from amazon.com) was fixed onto the middle of a piece of 3D knitted spacer mesh (12" x 12", 100% polyester, DNB59 from Apex Mills). The fabric was placed into a large Buchner funnel (10" in diameter) and a solution (250mL) of fibrillated, cross-linked, and fat liquored collagen from Example 1 was poured onto the fabric inside the embroidery hoop and vacuum was applied (5 inHg). The fabric andbiofabricated material were removed from the Buchner funnel, the hoop was removed and the material was dried for 24 hours at room temperature. A visual inspection showed the biofabricated leather material to be fully integrated with the spacer fabric, and that it has plugged the holes in the mesh where it had been applied.

### Example 11

A piece of cotton knitted jersey (purchased from Whaleys of Bradford) and a piece of polyester 3D knitted spacer (purchased from Apex Textiles) were both cut into rectangles measuring 3" x 5". These pieces of fabric were laid next to each other with a 1" gap between them. A rubber mask was then created by cutting a piece of silicone rubber (1/16 of an inch thick, with adhesive backing, purchased from McMaster Carr) into a rectangle measuring 5" x 7", a rectangular hole was then cut out measuring 4" x 5". The adhesive backing was removed and the mask was placed over the fabric pieces so that ½" of each was uncovered. The biofabricated paste (detailed below) was then applied, using a metal palette knife, until the hole in the mask was smoothly filled up to the top of the rubber. The sample was then dried at room temperature for 24 hours.

The fibrillated, cross-linked, and fat liquored collagen paste was made by dissolving 10g of collagen in 1L of water with 0.1 N HCl and was stirred overnight at 500 rpm. The pH was adjusted to 7.0 by adding 1 part 10x PBS to 9 parts collagen by weight and the solution was stirred at 500 rpm for 3 hours. 10% tanning agent (by weight of collagen) was added and mixed for 20 mins. The pH was adjusted to 8.5 by adding 20% sodium carbonate and the solution was stirred overnight at 500 rpm. The following day, the fibrils were washed twice in a centrifuge and re-suspended to the proper volume and mixed at 350 rpm. Then the pH was adjusted to 7.0 with 10% formic acid. 100% Hystretch v60 resin (by weight of collagen) was added and mixed for 30 mins. 100% offer of 20% fatliquor (by weight of collagen) was added and mixed for 30 mins. 10% microspheres (by weight of collagen) and 10% white pigment (by weight of collagen) was added and the pH was adjusted to 4.5 with 10% formic acid. Lastly, the solution was filtered and stirred each time the weight of the filtrate reached 50% of the weight of the solution, three times. 100g of this final dough was then weighed out and added to 100g of a textile binder (AquaBrite Black, purchased from Holden's Screen Supply) and mixed for 1 hour on a caframo mixer. The final concentration of solids is 10%, or one part solids to 9 parts water.

### Example 12

Two pieces of cotton knitted jersey (purchased from Whaleys of Bradford) were cut into rectangles measuring 12cm x 6cm. These pieces of fabric were laid next to each other with a 1cm gap between them. A rubber mask was then created by cutting a piece of silicone rubber (1/16" thick, with adhesive backing, purchased from McMaster Carr) into a rectangle measuring 16cm x 7.5cm, a rectangular hole was then cut out measuring 12cm x 3cm. The adhesive backing was removed and the mask was placed over the fabric pieces so that 1cm of each was uncovered. The biofabricated paste (detailed below) was then applied, using a metal palette knife, until the hole in the mask was smoothly filled up to the top of the rubber. The sample was then dried for 24 hours at 23°C. The sample was then tested with a 180 degree t-peel test on an Instron machine, and gave a reading of 0.7973N/mm. The fibrillated, cross-linked, and fat liquored collagen paste was made by dissolving 10g of collagen in 1L of water with 0.1 N HCl and was stirred overnight at 500 rpm. The pH was adjusted to 7.0 by adding 1 part 10x PBS to 9 parts collagen by weight and the solution was stirred at 500 rpm for 3 hours. 10% tanning agent (by weight of collagen) was added and mixed for 20 mins. The pH was adjusted to 8.5 by adding 20% sodium carbonate and the solution was stirred overnight at 500 rpm. The following day, the fibrils were washed twice in a centrifuge and re-suspended to the proper volume and mixed at 350 rpm. Then the pH was adjusted to 7.0 with 10% formic acid. 100% Hystretch v60 (by weight of collagen) was added and mixed for 30 mins. 100% offer of 20% fatliquor (by weight of collagen) was added and mixed for 30 mins.

10% microspheres (by weight of collagen) and 10% white pigment (by weight of collagen) was added and the pH was adjusted to 4.5 with 10% formic acid. Lastly, the solution was filtered and stirred each time the weight of the filtrate reached 50% of the weight of the solution, three times.

100g of this final dough was then weighed out and added to 100g of a textile binder (AquaBrite Puff Additive, purchased from Holden's Screen Supply) and mixed for 1 hour on a caframo mixer. The final concentration of solids is 10%, or one part solids to 9 parts water.

### Example 13

A piece of fabric (4" x 4", Lyocell 50% & Organic Cotton 50%, purchased from Simplifi Fabric) was masked off with rubber (1/16th" thick, purchased from McMaster Carr) leaving a 2" x 2" square piece of fabric exposed. A thin layer of the biofabricated paste from Example 11 was applied to the fabric. Then a dried piece of the biofabricated material from Example 1 was ground into fine particles using a handheld dremel. This 'leather flock' was then applied over half of the wet paste from Example 11. The sample dried overnight, at 23°C. The powder was bonded to the biofabricated leather such that it was not easily removed by hand.

### Example 14

A square piece of cotton knitted jersey (purchased from Whaleys of Bradford) measuring 3"x3" was cut out and had a silicone rubber stencil laid on top. The stencil was made from 1/16th thick adhesive backed silicone (purchased from McMaster Carr) and was cut into a square measuring 4"x4" with a 2" square hole cut into the middle. Then a layer of white wet paste from example 12 was applied to the surface of fabric in the hole and smoothed out using a palette knife. Then small amounts of black wet paste from example 11 were dropped into the white paste randomly using a spatula blade. Then both colours were mixed together to form a marbled pattern using a domestic hand sewing needle tip. The sample was then dried for 24 hours at room temperature.

### Example 15

A square piece of cotton knitted jersey (purchased from Whaleys of Bradford) measuring 5"x5" was cut out and had a silicone rubber stencil laid on top. The stencil was made from 1/16th thick adhesive backed silicone (purchased from McMaster Carr) and was cut into a square measuring 6"x6" with a 4" square hole cut into the middle. Then a layer of the wet paste from example 12 was applied to the surface of fabric in the hole and smoothed out using a palette knife. Then four squares of balsa wood (1/32nd" thick, purchased from Hobby Lobby) measuring ½" x ½" were cut out and embedded within the wet paste. The sample was then dried at room temperature for 24 hours. The biofabricated leather material had attached to the wood holding it in place, this technique is similar to marquetry but the secondary material was attached in place without creating a hole or using any additional adhesives.

### Example 16

A sphere measuring 1.5" in diameter was 3D printed using a photopolymer resin (purchased from formlabs.com) on a Formlabs 1 machine. The sphere had a leather grain texture on the surface, created during the printing. The dried, solid, sphere was then dipped into the wet paste from example 11, and left to dry at room temperature. Once dry the sphere was dipped again and dried in the same way. The result was a seamlessly coated 3-dimensional shape, which was made using no additional adhesives.

### Example 17

A fibrillated, cross-linked, and fat liquored collagen paste was made by dissolving 10g of collagen in 1L of water with 0.1N HCl and was stirred overnight at 500 rpm. The pH was adjusted to 7.0 by adding 1 part 10x PBS to 9 parts collagen by weight and the solution was stirred at 500 rpm for 3 hours. 10% tanning agent (by weight of collagen) was added and mixed for 20 mins. The pH was adjusted to 8.5 by adding 20% sodium carbonate and the solution was stirred overnight at 500 rpm. The following day, the solution was centrifuged and the precipitate was washed twice and re-suspended to a total volume half of the original volume and mixed at 350 rpm. The pH was adjusted to 7.0 with 10% formic acid. 100% Hystretch v60 resin (by weight of collagen) was added and mixed for 30 mins. 50% offer of 20% fatliquor (by weight of collagen) was added and mixed for 30 mins. 10% microspheres (by weight of collagen) and 10% white pigment (by weight of collagen) was added and the pH was adjusted to 4.0 with 10% formic acid. Lastly, the solution was filtered until ~8% solids remain creating a paste. A mixture is made of 2 parts paste and 1 part Hystretch v60 (or Puff binder) based on the solids of each component. The final solids of the mixture is ~10%. This mixture was hand mixed and applied on a 15 cm x15 cm x 1/8" thick mold on a cut out of black needle punched PET fabric (PE-03-020-60 from Sutherland Felt Company). The solution applied onto the fabric was then dried at 38C until the moisture content reaches ~300% (from its original 900%) and the mold was removed. The solution on top of the fabric was then hand rolled to pre-impregnate the paste into the fabric. Then, the partially integrated textile was laid between two 15x15cm steel plates and placed in the hot press (from Carver) pre-set to 60°C, where it was pressed at 6,000 psi for 10minutes. The sample was removed and allowed to finish drying overnight. The excess fabric that wasn't integrated with paste was then trimmed.

### Example 18

A piece of white, woven, silk crepe fabric (purchased from Whaleys Bradford Fabrics) was cut in a 6.5 x 6.5" square. The fabric was adhered to a flat sheet of cardboard propped up vertically with 505 temporary fabric spray adhesive on the back. A square of sheet, acetate material was cut larger than the area of the fabric and in the center, a circle with a 4" diameter was cut out, creating a stencil. The stencil was centered on top of the fabric and lightly adhered to the fabric with Odif Usa 505 spray and fix temporary fabric adhesive sprayed on the back of the stencil, thus exposing only a 4" diameter circle of fabric. A solution (100 mL) of fibrillated, cross-linked, and fat liquored collagen was poured into an MA-200 200 mL metal atomizer. The end of the metal atomizer's pipe was attached to a small, hand powered bellow. While one hand was used to hold the atomizer level, about 10" away from the fabric and stencil, the other hand repeatedly pressed the bellow so the solution sprayed out onto the exposed fabric. The solution was sprayed until the circle of fabric was opaque and completely covered with the solution. The fabric was left to air dry which took about a few hours. After dry, the stencil was removed from the surface of the fabric and a circle of solution adhered to the fabric remained.

The fibrillated, cross-linked, and fat liquored collagen solution was made by dissolving 10g collagen in 0.1N HCl solution with 1000mL DI water at 10 mg/mL and was stirred at 350 rpm for 4 hours. The pH was adjusted to 7.2 by adding 1 part pH 11.2 PBS to 9 parts collagen by weight and the solution was stirred at 500 rpm for 4 hours. 10% gluteraldehyde (by weight of collagen) was added, the pH was adjusted to 8 using 20% sodium carbonate. After an overnight reaction mixing at 450 rpm, the solution was centrifuged at 9000 rpm for 5 minutes at 20 C. The solution was resuspended with the same volume of DI water mixed at 250 rpm for 30 minutes. A 40% solution of Truposol Ben and Truposis G was made and 50% of that solution (by weight of collagen) added to the resuspended solution and mixed for 10 minutes. 10% microspheres (by weight of collagen) added and mixed for 30 minutes. The pH was lowered to 4.5 with 10% formic acid. Then 100% Hycar26652 (by weight of collagen) was added and mixed for 30 mins. After 30 minutes of mixing, the hand held proctor silex electric shearing blade was used to further break up the solution for 2 minutes. The solution was then mixed with a shearing impellor blade for 10 minutes. Lastly, the solution was poured into a Buchner funnel with filter paper (Whatman Grade 1 filter paper, purchased from Sigma Aldrich) in it, attached to a vacuum and vacuum filtered so the filtrate weight was 33.25% of the total solution weight.

### Example 19

A piece of fabric (15" by 15", of 0.5mm black needle-punched 100% polyester felt, purchased from Sutherland Felt Company) was cut. A 15" by 15" piece of acetate (Grafix Clear .003 Dura-Lar 40-Inch-by-50-Feet, Roll) was cut and pinned to a sheet of cardboard. A solution of fibrillated, cross-linked, and fat liquored collagen described below was poured into an MA-200 200 mL metal atomizer. The end of the metal atomizer's pipe was attached to a small, hand powered bellow. While one hand was used to hold the atomizer level, about 10" away from the acetate, the other hand repeatedly pressed the bellow so the solution sprayed out onto the acetate. The solution was sprayed until the acetate was opaque and completely covered with the solution. The acetate was left to air dry for 1 to 4 hours. After the acetate was completely dry, the process of spraying the solution of fibrillated, cross-linked, and fat liquored collagen was repeated two additional times. After the solution was dry, 505 fabric adhesive was sprayed onto the acetate with the dry solution of the fibrillated, cross-linked, and fat liquored collagen and adhered to a piece of fabric. The fabric with the acetate, adhesive, and the solution of fibrillated, cross-linked, and fat liquored collagen was placed between two metal plates. The plates were placed in a carver press at 60C for 10 minutes with 6 metric tons of pressure. The plates were removed from the press and the acetate was removed from the fabric transferring the solution of fibrillated, cross-linked, and fat-liquored collagen to the fabric.

The solution of fibrillated, cross-linked, and fat liquored collagen solution was made by dissolving 10g collagen in 0.1N HCl solution at 10 mg/mL and was stirred at 600 rpm for 3 hours. The pH was adjusted to 7.2 by adding 1 part 10x PBS pH 11.2 to 9 parts collagen by weight. The solution was stirred at 500rpm for 3 hours. 10% offer by weight of collagen of gluteraldehyde was added, the pH was adjusted to 8 using 20% sodium carbonate. After an overnight reaction mixing at 500 rpm, the solution was centrifuged at 9000 rpm for 5 minutes at 20C. The solution was resuspended to the same volume with deionized water and mixed at 250 rpm for 30 minutes. The pH was adjusted to 7.2 using 10% formic acid and mixed at 350 rpm. A 100mL glass bottle with deionized water was placed in a water bath at 50C. A 40% solution of Truposol Ben and Truposis G was made with the 50C water. A 50% offer by weight of collagen of the 40% Truposol Ben and Truposis G solution was added to the resuspended solution and mixed for 10 minutes. 10% offer by weight of collagen of microspheres was added to resuspended solution and mixed for 30 minutes. The pH was lowered to 4.0 with 10% formic acid and mixed for 30 minutes. A 100% offer by weight of collagen of Hycar 26552 was added to the resuspended solution and mixed for 30 minutes.

### Example 20

200 mL of a fibrillated, cross-linked, and fat liquored collagen solution was poured into a Buchner funnel with filter paper (Whatman Grade 1 filter paper, purchased from Sigma Aldrich), attached to a vacuum and vacuum filtered for 4 to 10 hours. An additional 200 mL of solution was poured into the Buchner funnel once the initial amount filtered into a cake. The sample was air dried for 1 to 3 days and then placed into the dehydrator (38C) for an additional 1 to 3 days. The final weight of the cake was between 8 to 10g with a height of 1cm and width of 3.5cm.

The cake was stabilized in a Bridgeport mill using a 4 flute 3/8 diameter end mill at approx. 300 rpm and shaped it into a block by subtracting material evenly one edge at a time. The cake was then sanded with a JET sanding belt with a 120 grit paper, one edge at a time to refine the edges. A Dremel was used to clean up the edges resulting in a block that was smooth on all side with a height of 1.5 inches, a length of 0.3 inch and a width of 1 inch.

The solution of fibrillated, cross-linked, and fat liquored collagen solution was made by dissolving 10g collagen in 0.1N HCl solution at 10 mg/mL and was stirred at 600 rpm for 3 hours. The pH was adjusted to 7.2 by adding 1 part 10x PBS pH 11.2 to 9 parts collagen by weight. The solution was stirred at 500rpm for 3 hours. 10% offer by weight of collagen of gluteraldehyde was added, the pH was adjusted to 8 using 20% sodium carbonate. After an overnight reaction mixing at 500 rpm, the solution was centrifuged at 9000 rpm for 5 minutes at 20C. The solution was resuspended to the same volume with deionized water and mixed at 250 rpm for 30 minutes. The pH was adjusted to 7.2 using 10% formic acid and mixed at 350 rpm. A 100mL glass bottle with deionized water was placed in a water bath at 50C. A 40% solution of Truposol Ben and Truposis G was made with the 50C water. A 50% offer by weight of collagen of the 40% Truposol Ben and Truposis G solution was added to the resuspended solution and mixed for 10 minutes. 10% offer by weight of collagen of microspheres was added to resuspended solution and mixed for 30 minutes. The pH was lowered to 4.0 with 10% formic acid and mixed for 30 minutes. A 100% offer by weight of collagen of Hycar 26552 was added to the resuspended solution and mixed for 30 min. After 30 minutes of mixing, 200mL of solution was poured into a Buchner funnel with filter paper (Whatman Grade 1 filter paper, purchased from Sigma Aldrich), attached to a vacuum and vacuum filtered for 4 to 10 hours. An additional 200mL of solution was poured into the Buchner funnel once the initial amount filtered into a cake. The sample was air dried for 1 to 3 days and then placed into the dehydrator for an additional 1 to 3 days. The final weight of the cake was 8 to 10g with a height of 1cm and width of 3.5cm.

### Example 21

A fibrillated, cross-linked, and fat liquored collagen paste is made by dissolving 10g of collagen in 1L of water with 0.1N HCl and is stirred overnight at 500 rpm. The pH is adjusted to 7.0 by adding 1 part 10x PBS to 9 parts collagen by weight and the solution is stirred at 500 rpm for 3 hours. 10% tanning agent (by weight of collagen) is added and mixed for 20 mins. The pH is adjusted to 8.5 by adding 20% sodium carbonate and the solution is stirred overnight at 500 rpm. The following day, the solution is centrifuged and the precipitate is washed twice and re-suspended to a total volume half of the original volume and mixed at 350 rpm. The pH is adjusted to 7.0 with 10% formic acid. 100% Hystretch v60 resin (by weight of collagen) is added and mixed for 30 mins. 50% offer of 20% fatliquor (by weight of collagen) is added and mixed for 30 mins. 10% microspheres (by weight of collagen) and 10% white pigment (by weight of collagen) is added and the pH is adjusted to 4.0 with 10% formic acid. Lastly, the solution is filtered until ~8% solids remain creating a paste. A mixture is hand mixed with 2 parts paste and 1 part Hystretch v60 (or Puff binder) based on the solids of each component. The final solids of the mixture is ~10%. The mixture is applied to the cavity of a rubber mold that can be cut into any shape with a thickness of 1/8" and placed on any type of fabric. The mixture is dried at 38°C until the moisture content reaches ~300% (from its original 900%) and the mold is removed. A stencil in the same shape as the rubber mold but 2 mm larger on all sides is cut out from a sheet of acetate (Grafix Clear .003 Dura-Lar 40-Inch-by-50-Feet, Roll). The stencil is sprayed with 505 temporary fabric spray adhesive on the back and then adhered to the fabric with the cut out area of the stencil surrounding the mixture applied to the fabric creating an acetate fabric. The acetate fabric is sandwiched between two sheets of Teflon and then sandwiched between two sheets of paper towels on top of the Teflon creating a sandwiched acetate fabric. The sandwiched acetate fabric is hand rolled to pre-impregnate the mixture into the fabric. Then, the sandwiched acetate fabric is laid between two 15x15cm steel plates and placed in the hot press (Carver) pre-set to 60°C, where it is pressed at 6,000 psi for 10 minutes. The sandwiched acetate fabric is removed from the press and allowed to cool and finish drying overnight. The paper towels and Teflon sheets are removed. The acetate stencil is removed if the sample is not being finished with finishing chemicals or kept on the fabric if the sample is going to be finished with finishing chemicals.

### Example 22

The solution of fibrillated, cross-linked, and fat liquored collagen is made by dissolving 10g collagen in 0.1N HCl solution at 10 mg/mL and is stirred at 600 rpm for 3 hours. The pH is adjusted to 7.2 by adding 1 part 10x PBS pH 11.2 to 9 parts collagen by weight. The solution is stirred at 500rpm for 3 hours. 10% offer by weight of collagen of gluteraldehyde is added, the pH is adjusted to 8 using 20% sodium carbonate. The solution is mixed overnight at 500 rpm, then the solution is centrifuged at 9,000 rpm for 5 minutes at 20°C. Precipitate at the bottom of the solution is removed. The solution is resuspended to the same volume (1000mL) with deionized water and mixed at 250 rpm for 30 minutes. The pH is adjusted to 7.2 using 10% formic acid and mixed at 350 rpm. A 100mL glass bottle with deionized water is placed in a water bath at 50°C. A solution of 20% Truposol Ben and 20% of Truposis G is made with the 50°C water. A 50% offer by weight of collagen of the Truposol Ben and Truposis G solution is added to the resuspended solution and mixed for 10 minutes. 10% offer by weight of collagen of microspheres is added to resuspended solution and mixed for 30 minutes. The pH is lowered to 4.0 with 10% formic acid and mixed for 30 minutes. A 100% offer by weight of collagen of Hycar 26552 is added to the resuspended solution and mixed for 30 min creating a collagen solution. 200mL of the collagen solution is poured into a Buchner funnel with a filter paper (Whatman Grade 1 filter paper, purchased from Sigma Aldrich) attached to a vacuum and vacuum filtered for 4 to 10 hours to create a collagen material. After filtering for 4 to 10 hours, a desired amount of a second material such as gold filings, wood shavings, iron filings and silver shavings are sprinkled on top of the vacuumed collagen material to a desired height. An additional 200mL of collagen solution is poured into the Buchner funnel on top of the second material covering the vacuumed collagen material. The sample is air dried for 1 to 3 days and then placed into the dehydrator for an additional 1 to 3 days creating a cake. The final weight of the cake is 8 to 10g plus the weight of the gold filings or other material referenced above.

The cake is stabilized in a Bridgeport mill using a 4 flute 3/8 diameter end mill at approx. 300 rpm and shaped into a block by subtracting material evenly one edge at a time. While subtracting material off the cake, gold filings or other secondary materials referenced above, are revealed as being layered in the center of the block as a continuous stripe going around the sides of the block. The collagen material holds the gold filings, suspending and adhering them together in a densely compacted, solid form. Using a Bridgeport mill to subtract material off the edges and top of the block, the amount of suspended gold filings layered within the collagen material that is seen on the exterior of the block, is controlled. The color and aesthetic visual of the block is thus changed by this process. The block is then sanded with a JET sanding belt with a 120 grit paper, one edge at a time to refine the edges. A Dremel is used to clean up the edges.

## Claims

1. A method of applying a collagen solution to a substrate, the method comprising: spraying a collagen solution onto a sheet of acetate forming a collagen coated side of the acetate sheet; drying the collagen; spraying an adhesive onto the collagen; placing a substrate onto the collagen coated side of the acetate sheet; placing the substrate and the collagen coated acetate sheet in a heated press; pressing the substrate and the collagen coated acetate sheet; cooling the substrate and the collagen coated acetate sheet; and removing the acetate sheet to produce a biofabricated material.

2. The method of claim 1, wherein collagen solution comprises recombinant bovine collagen.

3. The method of claim 1, wherein the adhesive is selected from the group consisting of polyurethanes, acrylics, epoxies and combinations thereof.

4. The method of claim 1, wherein the substrate is selected from the group consisting of fabrics, metals, wood, plastics, yarn, fibers, 3D printed structures, walls, concrete, skin and leather.

5. The method of claim 1, wherein the pressure of the heated press ranges from 1 to 11 metric tons.

6. The method of claim 1, wherein the temperature of the heated press ranges from 25 to 190°C.

7. The method of claim 1, wherein the substrate is pressed from 1 to 30 minutes.

## Patentansprüche

1. Verfahren zum Aufbringen einer Kollagenlösung auf ein Substrat, wobei das Verfahren Folgendes umfasst: Sprühen einer Kollagenlösung auf eine Bahn aus Acetat, wodurch eine mit Kollagen beschichtete Seite der Acetatbahn gebildet wird; Trocknen des Kollagens; Sprühen eines Klebstoffs auf das Kollagen; Anordnen eines Substrats auf der mit Kollagen beschichteten Seite der Acetatbahn; Anordnen des Substrats und der mit Kollagen beschichteten Acetatbahn in einer beheizten Presse; Pressen des Substrats und der mit Kollagen beschichteten Acetatbahn; Abkühlen des Substrats und der mit Kollagen beschichteten Acetatbahn ; und Entnehmen der Acetatbahn zum Herstellen eines biotechnologisch hergestellten Materials.

2. Verfahren nach Anspruch 1, wobei die Kollagenlösung rekombinantes Rinderkollagen umfasst.

3. Verfahren nach Anspruch 1, wobei der Klebstoff ausgewählt ist aus der Gruppe bestehend aus Polyurethanen, Acrylaten, Epoxiden und Kombinationen daraus.

4. Verfahren nach Anspruch 1, wobei das Substrat ausgewählt ist aus der Gruppe bestehend aus Stoffen, Metallen, Holz, Kunststoffen, Garn, Fasern, 3D-Druckstrukturen, Wänden, Beton, Haut und Leder.

5. Verfahren nach Anspruch 1, wobei der Druck der beheizten Presse in einem Bereich von 1 bis 11 metrischen Tonnen liegt.

6. Verfahren nach Anspruch 1, wobei die Temperatur der beheizten Presse in einem Bereich von 25 bis 190 °C liegt.

7. Verfahren nach Anspruch 1, wobei das Substrat 1 bis 30 Minuten lang gepresst wird.

## Revendications

1. Procédé d'application d'une solution de collagène à un substrat, le procédé comprenant : la pulvérisation d'une solution de collagène sur une feuille d'acétate formant un côté revêtu de collagène de la feuille d'acétate ; le séchage du collagène ; la pulvérisation d'un adhésif sur le collagène ; le placement d'un substrat sur le côté revêtu de collagène de la feuille d'acétate ; le placement du substrat et de la feuille d'acétate revêtue de collagène dans une presse chauffée ; le pressage du substrat et de la feuille d'acétate revêtue de collagène ; le refroidissement du substrat et de la feuille d'acétate revêtue de collagène ; et l'enlèvement de la feuille d'acétate pour produire un matériau biofabriqué.

2. Procédé selon la revendication 1, dans lequel la solution de collagène comprend du collagène bovin recombinant.

3. Procédé selon la revendication 1, dans lequel l'adhésif est choisi dans le groupe constitué par les polyuréthanes, les acryliques, les époxys et leurs combinaisons.

4. Procédé selon la revendication 1, dans lequel le substrat est choisi dans le groupe constitué par les tissus, les métaux, le bois, les plastiques, un fil, les structures imprimées en 3D, les parois, le béton, la peau et le cuir.

5. Procédé selon la revendication 1, dans lequel la pression de la presse chauffée se situe dans la plage allant de 1 à 11 tonnes métriques.

6. Procédé selon la revendication 1, dans lequel la température de la presse chauffée se situe dans la plage allant de 25 à 190 °C.

7. Procédé selon la revendication 1, dans lequel le substrat est pressé durant 1 à 30 minutes.
